# EUROPÄISCHE PATENTSCHRIFT

(11) **EP 0 935 664 B1**
(45) Veröffentlichungstag und Bekanntmachung des Hinweises auf die Patenterteilung: **24.06.2009**
(21) Anmeldenummer: 97944777.8
(22) Anmeldetag: 21.08.1997
(51) Int. Cl.: C12N 15/73, C12N 15/10, A61K 39/02

(54) **NEUE SYSTEME ZUR REGULATION DER GENEXPRESSION**
NEW SYSTEMS FOR REGULATING GENETIC EXPRESSION
NOUVEAUX SYSTEMES DE REGULATION DE L'EXPRESSION GENETIQUE

(30) Priorität: 21.08.1996 DE 19633698
(43) Veröffentlichungstag der Anmeldung: 18.08.1999
(73) Patentinhaber: EVAX Technologies AG, 82152 Martinsried/München (DE)
(72) Erfinder: LUBITZ, Werner, A-1080 Wien (AT); JECHLINGER, Wolfgang, A-1080 Wien (AT); SZOSTAK, Michael, A-2344 Maria Enzersdorf (AT); WITTE,Angela, a-1020 Wien (AT)
(74) Vertreter: Weiss, Wolfgang
(86) Internationale Anmeldenummer: PCT/EP1997/004560
(87) Internationale Veröffentlichungsnummer: WO 1998/007874

(56) Entgegenhaltungen:
- WO-A-91/13155
- HERSHBERGER P.A.: "Interference by PR-bound RNA polymerase with PRM function in vitro." JOURNAL OF BIOLOGICAL CHEMISTRY, Bd. 268, Nr. 12, 25.April 1993, Seiten 8943-8948, XP002052321
- KUNKEL T. A.: "RAPID AND EFFICIENT SITE-SPECIFIC MUTAGENESIS WITHOUT PHENOTYPIC SELECTION" PROCEEDINGS OF THE NATIONAL ACADEMY OF SCIENCES OF THE USA, Bd. 82, Januar 1985, Seiten 488-492, XP002052322 in der Anmeldung erwähnt
- SZOSTAK M P ET AL: "Bacterial ghosts: non-living candidate vaccines" JOURNAL OF BIOTECHNOLOGY, Bd. 44, Nr. 1, 26.Januar 1996, Seite 161-170 XP004036862

### SEQUENZPROTOKOLL

(1) ALLGEMEINE ANGABEN:
   (i) ANMELDER:
      (A) NAME: Prof. Dr. Werner Lubitz
      (B) STRASSE: Schoenborngasse 12/7
      (C) ORT: Wien
      (E) LAND: Oesterreich
      (F) POSTLEITZAHL: 1080
   (ii) BEZEICHNUNG DER ERFINDUNG: Neue Systeme zur Regulation der Genexpression
   (iii) ANZAHL DER SEQUENZEN: 10
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 82 Basenpaare
      (B) ART: Nucleotid
      (C) STRANGFORM: beides
      (D) TOPOLOGIE: linear
   (vi) URSPRÜNGLICHE HERKUNFT:
      (A) ORGANISMUS: Lambda-OR-Operator (Wildtyp)
   (xi) SEQUENZBESCHREIBUNG: SEQ ID NO: 1:
   ACGTTAAATC TATCACCGCA AGGGATAAAT ATCTAACACC GTGCGTGTTG ACTATTTTAC    60
   CTCTGGCGGT GATAATGGTT GC                                             82
(2) ANGABEN ZU SEQ ID NO: 2:
   (i) SEQUENZKENNZEICHEN:
      (A) LÄNGE: 82 Basenpaare
      (B) ART: Nucleotid
      (C) STRANGFORM: beides
      (D) TOPOLOGIE: linear
   (vi) URSPRÜNGLICHE HERKUNFT:
      (A) ORGANISMUS: Lambda-OR-Operator (Mutante)
   (xi) SEQUENZBESCHREIBUNG: SEQ ID NO: 2:
   ACGTTAAATC TATCACCGCA AGGGATAAAT ATCTAACACC GCGCGTGTTG ACTATTTTAC    60
   CTCTGGCGGT GATAATGGTT GC                                             82
(2) ANGABEN ZU SEQ ID NO: 3:
   (i) SEQUENZKENNZEICHEN:
      (A) LÄNGE: 85 Basenpaare
      (B) ART: Nucleotid
      (C) STRANGFORM: beides
      (D) TOPOLOGIE: linear
   (vi) URSPRÜNGLICHE HERKUNFT:
      (A) ORGANISMUS: Lambda-OL-Operator (Wildtyp)
   (xi) SEQUENZBESCHREIBUNG: SEQ ID NO: 3:
   ACATACAGAT AACCATCTGC GGTGATAAAT TATCTCTGGC GGTGTTGACA TAAATACCAC     60
   TGGCGGTGAT ACTGAGCACA TCAGC                                           85
(2) ANGABEN ZU SEQ ID NO: 4:
   (i) SEQUENZKENNZEICHEN:
      (A) LÄNGE: 1601 Basenpaare
      (B) ART: Nucleotid
      (C) STRANGFORM: Doppelstrang
      (D) TOPOLOGIE: beides
   (vi) URSPRÜNGLICHE HERKUNFT:
      (A) ORGANISMUS: pAW12 Fragment
   (ix) MERKMAL:
      (A) NAME/SCHLÜSSEL: CDS
      (B) LAGE:complement (106..816)
   (ix) MERKMAL:
      (A) NAME/SCHLÜSSEL: CDS
      (B) LAGE:1144..1416
   (xi) SEQUENZBESCHREIBUNG: SEQ ID NO: 4:
   ATTTACTATG TTATGTTCTG AGGGGAGTGA AAATTCCCCT AATTCGATGA AGATTCTTGC 60
   TCAATTGTTA TCAGCTATGC GCCGACCAGA ACACCTTGCC GATCAGCCAA ACGTCTCTTC 120
   AGGCCACTGA CTAGCGATAA CTTTCCCCAC AACGGAACAA CTCTCATTGC ATGGGATCAT 180
   TGGGTACTGT GGGTTTAGTG GTTGTAAAAA CACCTGACCG CTATCCCTGA TCAGTTTCTT 240
   GAAGGTAAAC TCATCACCCC CAAGTCTGGC TATGCAGAAA TCACCTGGCT CAACAGCCTG 300
   CTCAGGGTCA ACGAGAATTA ACATTCCGTC AGGAAAGCTT GGCTTGGAGC CTGTTGGTGC 360
   GGTCATGGAA TTACCTTCAA CCTCAAGCCA GAATGCAGAA TCACTGGCTT TTTTGGTTGT 420
   GCTTACCCAT CTCTCCGCAT CACCTTTGGT AAAGGTTCTA AGCTTAGGTG AGAACATCCC 480
   TGCCTGAACA TGAGAAAAAA CAGGGTACTC ATACTCACTT CTAAGTGACG GCTGCATACT 540
   AACCGCTTCA TACATCTCGT AGATTTCTCT GGCGATTGAA GGGCTAAATT CTTCAACGCT 600
   AACTTTGAGA ATTTTTGTAA GCAATGCGGC GTTATAAGCA TTTAATGCAT TGATGCCATT 660
   AAATAAAGCA CCAACGCCTG ACTGCCCCAT CCCCATCTTG TCTGCGACAG ATTCCTGGGA 720
   TAAGCCAAGT TCATTTTTCT TTTTTTCATA AATTGCTTTA AGGCGACGTG CGTCCTCAAG 780
   CTGCTCTTGT GTTAATGGTT TCTTTTTTGT GCTCATACGT TAAATCTATC ACCGCAAGGG 840
   ATAAATATCT AACACCGCGC GTGTTGACTA TTTTACCTCT GGCGGTGATA ATGGTTGCAT 900
   GTACTAAGTA GGTTGTATGG AACAACGCAT AACCCTGAAA GATTATGCAA TGCGCTTTGG 960
   GCAAACCAAG ACAGCTAAAG ATCCTCTAGA GTCGACCTGC AGGCATGCAA GCTTATCGAA 1020
   TTCTCATTCA GGCTTCTGCC GTTTTGGATT TAACCGAAGA TGATTTCGAT TTTCTGACGA 1080
   GTAACAAAGT TTGGATTGCT ACTGACCGCT CTCGTGCTCG TCGCTGCGTT GAGGCTTGCG 1140
   TTT ATG GTA CGC TGG ACT TTG TGG GAT ACC CTC GCT TTC CTG CTC CTG 1188
   Met Val Arg Trp Thr Leu Trp Asp Thr Leu Ala Phe Leu Leu Leu
   1 5 10 15
   TTG AGT TTA TTG CTG CCG TCA TTG CTT ATT ATG TTC ATC CCG TCA ACA 1236
   Leu Ser Leu Leu Leu Pro Ser Leu Leu Ile Met Phe Ile Pro Ser Thr
   20 25 30
   TTC AAA CGG CCT GTC TCA TCA TGG AAG GCG CTG AAT TTA CGG AAA ACA 1284
   Phe Lys Arg Pro Val Ser Ser Trp Lys Ala Leu Asn Leu Arg Lys Thr
   35 40 45
   TTA TTA ATG GCG TCG AGC GTC CGG TTA AAG CCG CTG AAT TGT TCG CGT      1332
   Leu Leu Met Ala Ser Ser Val Arg Leu Lys Pro Leu Asn Cys Ser Arg
            50                  55                  60
   TTA CCT TGC GTG TAC GCG CAG GAA ACA CTG ACG TTC TTA CTG ACG CAG      1380
   Leu Pro Cys Val Tyr Ala Gln Glu Thr Leu Thr Phe Leu Leu Thr Gln
        65                  70                  75
   AAG AAA ACG TGC GTC AAA AAT TAC GTG CAG AAG GAG TGATGTAATG           1426
   Lys Lys Thr Cys Val Lys Asn Tyr Val Gln Lys Glu
    80                  85                  90
   TCTAAAGGTA AAAAACGTTC TGGCGCTCGC CCTGGTCGTC CGCAGCCGTT GCGAGGTACT    1486
   AAAGGCAAGC GTAAAGGCGC TCGTCTTTGG TATGTAGGTG GTCAACAATT TTAATTGCAG    1546
   GGGCTTCGGC CCTTACTTGA GGATAAATTA TGTCTAATAT TCAAACTGGC GCCGA         1601
(2) ANGABEN ZU SEQ ID NO: 5:
   (i) SEQUENZKENNZEICHEN:
      (A) LÄNGE: 237 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 5:
   Met Ser Thr Lys Lys Lys Pro Leu Thr Gln Glu Gln Leu Glu Asp Ala
    1               5                  10                  15
   Arg Arg Leu Lys Ala Ile Tyr Glu Lys Lys Lys Asn Glu Leu Gly Leu
               20                  25                  30
   Ser Gln Glu Ser Val Ala Asp Lys Met Gly Met Gly Gln Ser Gly Val
           35                  40                  45
   Gly Ala Leu Phe Asn Gly Ile Asn Ala Leu Asn Ala Tyr Asn Ala Ala
       50                  55                  60
   Leu Leu Thr Lys Ile Leu Lys Val Ser Val Glu Glu Phe Ser Pro Ser
   65                  70                  75                  80
   Ile Ala Arg Glu Ile Tyr Glu Met Tyr Glu Ala Val Ser Met Gln Pro
                   85                  90                  95
   Ser Leu Arg Ser Glu Tyr Glu Tyr Pro Val Phe Ser His Val Gln Ala
               100                 105                 110
   Gly Met Phe Ser Pro Lys Leu Arg Thr Phe Thr Lys Gly Asp Ala Glu
           115                 120                 125
   Arg Trp Val Ser Thr Thr Lys Lys Ala Ser Asp Ser Ala Phe Trp Leu
       130                 135                 140
   Glu Val Glu Gly Asn Ser Met Thr Ala Pro Thr Gly Ser Lys Pro Ser
   145                 150                 155                 160
   Phe Pro Asp Gly Met Leu Ile Leu Val Asp Pro Glu Gln Ala Val Glu
                   165                 170                 175
   Pro Gly Asp Phe Cys Ile Ala Arg Leu Gly Gly Asp Glu Phe Thr Phe
               180                 185                 190
   Lys Lys Leu Ile Arg Asp Ser Gly Gln Val Phe Leu Gln Pro Leu Asn
           195                 200                 205
   Pro Gln Tyr Pro Met Ile Pro Cys Asn Glu Ser Cys Ser Val Val Gly
       210                 215                 220
   Lys Val Ile Ala Ser Gln Trp Pro Glu Glu Thr Phe Gly
   225                 230                 235
(2) ANGABEN ZU SEQ ID NO: 6:
   (i) SEQUENZKENNZEICHEN:
      (A) LÄNGE: 91 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 6:
   Met Val Arg Trp Thr Leu Trp Asp Thr Leu Ala Phe Leu Leu Leu Leu
   1               5                   10                  15
   Ser Leu Leu Leu Pro Ser Leu Leu Ile Met Phe Ile Pro Ser Thr Phe
               20                  25                  30
   Lys Arg Pro Val Ser Ser Trp Lys Ala Leu Asn Leu Arg Lys Thr Leu
           35                  40                  45
   Leu Met Ala Ser Ser Val Arg Leu Lys Pro Leu Asn Cys Ser Arg Leu
       50                  55                  60
   Pro Cys Val Tyr Ala Gln Glu Thr Leu Thr Phe Leu Leu Thr Gln Lys
   65                  70                  75                  80
   Lys Thr Cys Val Lys Asn Tyr Val Gln Lys Glu
                   85                  90
(2) ANGABEN ZU SEQ ID NO: 7:
   (i) SEQUENZKENNZEICHEN:
      (A) LÄNGE: 2834 Basenpaare
      (B) ART: Nucleotid
      (C) STRANGFORM: Doppelstrang
      (D) TOPOLOGIE: beides
   (vi) URSPRÜNGLICHE HERKUNFT:
      (A) ORGANISMUS: pCSJ Fragment
   (ix) MERKMAL:
      (A) NAME/SCHLÜSSEL: CDS
      (B) LAGE:complement (106..816)
   (ix) MERKMAL:
      (A) NAME/SCHLÜSSEL: CDS
      (B) LAGE:1025..2104
   (ix) MERKMAL:
      (A) NAME/SCHLÜSSEL: CDS
      (B) LAGE:2377..2649
   (xi) SEQUENZBESCHREIBUNG: SEQ ID NO: 7:
   ATTTACTATG TTATGTTCTG AGGGGAGTGA AAATTCCCCT AATTCGATGA AGATTCTTGC    60
   TCAATTGTTA TCAGCTATGC GCCGACCAGA ACACCTTGCC GATCAGCCAA ACGTCTCTTC   120
   AGGCCACTGA CTAGCGATAA CTTTCCCCAC AACGGAACAA CTCTCATTGC ATGGGATCAT   180
   TGGGTACTGT GGGTTTAGTG GTTGTAAAAA CACCTGACCG CTATCCCTGA TCAGTTTCTT   240
   GAAGGTAAAC TCATCACCCC CAAGTCTGGC TATGCAGAAA TCACCTGGCT CAACAGCCTG   300
   CTCAGGGTCA ACGAGAATTA ACATTCCGTC AGGAAAGCTT GGCTTGGAGC CTGTTGGTGC   360
   GGTCATGGAA TTACCTTCAA CCTCAAGCCA GAATGCAGAA TCACTGGCTT TTTTGGTTGT   420
   GCTTACCCAT CTCTCCGCAT CACCTTTGGT AAAGGTTCTA AGCTTAGGTG AGAACATCCC   480
   TGCCTGAACA TGAGAAAAAA CAGGGTACTC ATACTCACTT CTAAGTGACG GCTGCATACT   540
   AACCGCTTCA TACATCTCGT AGATTTCTCT GGCGATTGAA GGGCTAAATT CTTCAACGCT   600
   AACTTTGAGA ATTTTTGTAA GCAATGCGGC GTTATAAGCA TTTAATGCAT TGATGCCATT   660
   AAATAAAGCA CCAACGCCTG ACTGCCCCAT CCCCATCTTG TCTGCGACAG ATTCCTGGGA   720
   TAAGCCAAGT TCATTTTTCT TTTTTTCATA AATTGCTTTA AGGCGACGTG CGTCCTCAAG   780
   CTGCTCTTGT GTTAATGGTT TCTTTTTTGT GCTCATACGT TAAATCTATC ACCGCAAGGG   840
   ATAAATATCT AACACCGCGC GTGTTGACTA TTTTACCTCT GGCGGTGATA ATGGTTGCAT   900
   GTACTAAGTA GGTTGTATGG AACAACGCAT AACCCTGAAA GATTATGCAA TGCGCTTTGG   960
   GCAAACCAAG ACAGCTAAAG ATCCTCTAGA GCGCCCGGAA GAGAGTCAAT TCAGGGTGGT  1020
   GAAT GTG AAA CCA GTA ACG TTA TAC GAT GTC GCA GAG TAT GCC GGT GTC   1069
        Val Lys Pro Val Thr Leu Tyr Asp Val Ala Glu Tyr Ala Gly Val
                     95                  100                 105
   TCT TAT CAG ACC GTT TCC CGC GTG GTG AAC CAG GCC AGC CAC GTT TCT    1117
   Ser Tyr Gln Thr Val Ser Arg Val Val Asn Gln Ala Ser His Val Ser
               110                 115                 120
   GCG AAA ACG CGG GAA AAA GTG GAA GCG GCG ATG GCG GAG CTG AAT TAC      1165
   Ala Lys Thr Arg Glu Lys Val Glu Ala Ala Met Ala Glu Leu Asn Tyr
       125                 130                 135
   ATT CCC AAC CGC GTG GCA CAA CAA CTG GCG GGC AAA CAG TCG TTG CTG      1213
   Ile Pro Asn Arg Val Ala Gln Gln Leu Ala Gly Lys Gln Ser Leu Leu
       140                 145                 150
   ATT GGC GTT GCC ACC TCC AGT CTG GCC CTG CAC GCG CCG TCG CAA ATT      1261
   Ile Gly Val Ala Thr Ser Ser Leu Ala Leu His Ala Pro Ser Gln Ile
   155                 160                 165                 170
   GTC GCG GCG ATT AAA TCT CGC GCC GAT CAA CTG GGT GCC AGC GTG GTG      1309
   Val Ala Ala Ile Lys Ser Arg Ala Asp Gln Leu Gly Ala Ser Val Val
                   175                 180                 185
   GTG TCG ATG GTA GAA CGA AGC GGC GTC GAA GCC TGT AAA GCG GCG GTG      1357
   Val Ser Met Val Glu Arg Ser Gly Val Glu Ala Cys Lys Ala Ala Val
               190                 195                 200
   CAC AAT CTT CTC GCG CAA CGC GTC AGT GGG CTG ATC ATT AAC TAT CCG      1405
   His Asn Leu Leu Ala Gln Arg Val Ser Gly Leu Ile Ile Asn Tyr Pro
           205                 210                 215
   CTG GAT GAC CAG GAT GCC ATT GCT GTG GAA GCT GCC TGC ACT AAT GTT      1453
   Leu Asp Asp Gln Asp Ala Ile Ala Val Glu Ala Ala Cys Thr Asn Val
       220                 225                 230
   CCG GCG TTA TTT CTT GAT GTC TCT GAC CAG ACA CCC ATC AAC AGT ATT      1501
   Pro Ala Leu Phe Leu Asp Val Ser Asp Gln Thr Pro Ile Asn Ser Ile
   235                 240                 245                 250
   ATT TTC TCC CAT GAA GAC GGT ACG CGA CTG GGC GTG GAG CAT CTG GTC      1549
   Ile Phe Ser His Glu Asp Gly Thr Arg Leu Gly Val Glu His Leu Val
                   255                 260                 265
   GCA TTG GGT CAC CAG CAA ATC GCG CTG TTA GCG GGC CCA TTA AGT TCT      1597
   Ala Leu Gly His Gln Gln Ile Ala Leu Leu Ala Gly Pro Leu Ser Ser
               270                 275                 280
   GTC TCG GCG CGT CTG CGT CTG GCT GGC TGG CAT AAA TAT CTC ACT CGC      1645
   Val Ser Ala Arg Leu Arg Leu Ala Gly Trp His Lys Tyr Leu Thr Arg
           285                 290                 295
   AAT CAA ATT CAG CCG ATA GCG GAA CGG GAA GGC GAC TGG AGT GCC ATG      1693
   Asn Gln Ile Gln Pro Ile Ala Glu Arg Glu Gly Asp Trp Ser Ala Met
       300                 305                 310
   TCC GGT TTT CAA CAA ACC ATG CAA ATG CTG AAT GAG GGC ATC GTT CCC      1741
   Ser Gly Phe Gln Gln Thr Met Gln Met Leu Asn Glu Gly Ile Val Pro
   315                 320                 325                 330
   ACT GCG ATG CTG GTT GCC AAC GAT CAG ATG GCG CTG GGC GCA ATG CGC      1789
   Thr Ala Met Leu Val Ala Asn Asp Gln Met Ala Leu Gly Ala Met Arg
                   335                 340                 345
   GCC ATT ACC GAG TCC GGG CTG CGC GTT GGT GCG GAT ATC TCG GTA GTG      1837
   Ala Ile Thr Glu Ser Gly Leu Arg Val Gly Ala Asp Ile Ser Val Val
               350                 355                 360
   GGA TAC GAC GAT ACC GAA GAC AGC TCA TGT TAT ATC CCG CCG TCA ACC      1885
   Gly Tyr Asp Asp Thr Glu Asp Ser Ser Cys Tyr Ile Pro Pro Ser Thr
           365                 370                 375
   ACC ATC AAA CAG GAT TTT CGC CTG CTG GGG CAA ACC AGC GTG GAC CGC      1933
   Thr Ile Lys Gln Asp Phe Arg Leu Leu Gly Gln Thr Ser Val Asp Arg
       380                 385                 390
   TTG CTG CAA CTC TCT CAG GGC CAG GCG GTG AAG GGC AAT CAG CTG TTG      1981
   Leu Leu Gln Leu Ser Gln Gly Gln Ala Val Lys Gly Asn Gln Leu Leu
   395                 400                 405                 410
   CCC GTC TCA CTG GTG AAA AGA AAA ACC ACC CTG GCG CCC AAT ACG CAA      2029
   Pro Val Ser Leu Val Lys Arg Lys Thr Thr Leu Ala Pro Asn Thr Gln
                   415                 420                 425
   ACC GCC TCT CCC CGC GCG TTG GCC GAT TCA TTA ATG CAG CTG GCA CGA      2077
   Thr Ala Ser Pro Arg Ala Leu Ala Asp Ser Leu Met Gln Leu Ala Arg
               430                 435                 440
   CAG GTT TCC CGA CTG GAA AGC GGG CAG TGAGCGCAAC GCAATTAATG            2124
   Gln Val Ser Arg Leu Glu Ser Gly Gln
           445                 450
   TGAGTTAGCT CACTCATTAG GCACCCCAGG CTTTACACTT TATGCTTCCG GCTCGTATGT    2184
   TGTGTGGAAT TGTGAGCGGA TAACAATTTC ACACAGGAAA CAGCTCTGCA GGCATGCAAG    2244
   CTTATCGAAT TCTCATTCAG GCTTCTGCCG TTTTGGATTT AACCGAAGAT GATTTCGATT    2304
   TTCTGACGAG TAACAAAGTT TGGATTGCTA CTGACCGCTC TCGTGCTCGT CGCTGCGTTG    2364
   AGGCTTGCGT TT ATG GTA CGC TGG ACT TTG TGG GAT ACC CTC GCT TTC        2412
                 Met Val Arg Trp Thr Leu Trp Asp Thr Leu Ala Phe
                  1               5                  10
   CTG CTC CTG TTG AGT TTA TTG CTG CCG TCA TTG CTT ATT ATG TTC ATC      2460
   Leu Leu Leu Leu Ser Leu Leu Leu Pro Ser Leu Leu Ile Met Phe Ile
           15                  20                  25
   CCG TCA ACA TTC AAA CGG CCT GTC TCA TCA TGG AAG GCG CTG AAT TTA      2508
   Pro Ser Thr Phe Lys Arg Pro Val Ser Ser Trp Lys Ala Leu Asn Leu
       30                  35                  40
   CGG AAA ACA TTA TTA ATG GCG TCG AGC GTC CGG TTA AAG CCG CTG AAT      2556
   Arg Lys Thr Leu Leu Met Ala Ser Ser Val Arg Leu Lys Pro Leu Asn
   45                  50                  55                  60
   TGT TCG CGT TTA CCT TGC GTG TAC GCG CAG GAA ACA CTG ACG TTC TTA      2604
   Cys Ser Arg Leu Pro Cys Val Tyr Ala Gln Glu Thr Leu Thr Phe Leu
                   65                  70                  75
   CTG ACG CAG AAG AAA ACG TGC GTC AAA AAT TAC GTG CAG AAG GAG          2649
   Leu Thr Gln Lys Lys Thr Cys Val Lys Asn Tyr Val Gln Lys Glu
               80                  85                  90
   TGATGTAATG TCTAAAGGTA AAAAACGTTC TGGCGCTCGC CCTGGTCGTC CGCAGCCGTT    2709
   GCGAGGTACT AAAGGCAAGC GTAAAGGCGC TCGTCTTTGG TATGTAGGTG GTCAACAATT    2769
   TTAATTGCAG GGGCTTCGGC CCTTACTTGA GGATAAATTA TGTCTAATAT TCAAACTGGC    2829
   GCCGA                                                                2834
(2) ANGABEN ZU SEQ ID NO: 8:
   (i) SEQUENZKENNZEICHEN:
      (A) LÄNGE: 237 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 8:
   Met Ser Thr Lys Lys Lys Pro Leu Thr Gln Glu Gln Leu Glu Asp Ala
    1               5                  10                  15
   Arg Arg Leu Lys Ala Ile Tyr Glu Lys Lys Lys Asn Glu Leu Gly Leu
               20                  25                  30
   Ser Gln Glu Ser Val Ala Asp Lys Met Gly Met Gly Gln Ser Gly Val
           35                  40                  45
   Gly Ala Leu Phe Asn Gly Ile Asn Ala Leu Asn Ala Tyr Asn Ala Ala
       50                  55                  60
   Leu Leu Thr Lys Ile Leu Lys Val Ser Val Glu Glu Phe Ser Pro Ser
   65                  70                  75                  80
   Ile Ala Arg Glu Ile Tyr Glu Met Tyr Glu Ala Val Ser Met Gln Pro
                   85                  90                  95
   Ser Leu Arg Ser Glu Tyr Glu Tyr Pro Val Phe Ser His Val Gln Ala
               100                 105                 110
   Gly Met Phe Ser Pro Lys Leu Arg Thr Phe Thr Lys Gly Asp Ala Glu
           115                 120                 125
   Arg Trp Val Ser Thr Thr Lys Lys Ala Ser Asp Ser Ala Phe Trp Leu
       130                 135                 140
   Glu Val Glu Gly Asn Ser Met Thr Ala Pro Thr Gly Ser Lys Pro Ser
   145                 150                 155                 160
   Phe Pro Asp Gly Met Leu Ile Leu Val Asp Pro Glu Gln Ala Val Glu
                   165                 170                 175
   Pro Gly Asp Phe Cys Ile Ala Arg Leu Gly Gly Asp Glu Phe Thr Phe
               180                 185                 190
   Lys Lys Leu Ile Arg Asp Ser Gly Gln Val Phe Leu Gln Pro Leu Asn
           195                 200                 205
   Pro Gln Tyr Pro Met Ile Pro Cys Asn Glu Ser Cys Ser Val Val Gly
       210                 215                 220
   Lys Val Ile Ala Ser Gln Trp Pro Glu Glu Thr Phe Gly
   225                 230                 235
(2) ANGABEN ZU SEQ ID NO: 9:
   (i) SEQUENZKENNZEICHEN:
      (A) LÄNGE: 360 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 9:
   Val Lys Pro Val Thr Leu Tyr Asp Val Ala Glu Tyr Ala Gly Val Ser
    1               5                  10                  15
   Tyr Gln Thr Val Ser Arg Val Val Asn Gln Ala Ser His Val Ser Ala
               20                  25                  30
   Lys Thr Arg Glu Lys Val Glu Ala Ala Met Ala Glu Leu Asn Tyr Ile
           35                  40                  45
   Pro Asn Arg Val Ala Gln Gln Leu Ala Gly Lys Gln Ser Leu Leu Ile
       50                  55                  60
   Gly Val Ala Thr Ser Ser Leu Ala Leu His Ala Pro Ser Gln Ile Val
   65                  70                  75                  80
   Ala Ala Ile Lys Ser Arg Ala Asp Gln Leu Gly Ala Ser Val Val Val
                   85                  90                  95
   Ser Met Val Glu Arg Ser Gly Val Glu Ala Cys Lys Ala Ala Val His
               100                 105                 110
   Asn Leu Leu Ala Gln Arg Val Ser Gly Leu Ile Ile Asn Tyr Pro Leu
           115                 120                 125
   Asp Asp Gln Asp Ala Ile Ala Val Glu Ala Ala Cys Thr Asn Val Pro
       130                 135                 140
   Ala Leu Phe Leu Asp Val Ser Asp Gln Thr Pro Ile Asn Ser Ile Ile
   145                 150                 155                 160
   Phe Ser His Glu Asp Gly Thr Arg Leu Gly Val Glu His Leu Val Ala
                   165                 170                 175
   Leu Gly His Gln Gln Ile Ala Leu Leu Ala Gly Pro Leu Ser Ser Val
               180                 185                 190
   Ser Ala Arg Leu Arg Leu Ala Gly Trp His Lys Tyr Leu Thr Arg Asn
           195                 200                 205
   Gln Ile Gln Pro Ile Ala Glu Arg Glu Gly Asp Trp Ser Ala Met Ser
       210                 215                 220
   Gly Phe Gln Gln Thr Met Gln Met Leu Asn Glu Gly Ile Val Pro Thr
   225                 230                 235                 240
   Ala Met Leu Val Ala Asn Asp Gln Met Ala Leu Gly Ala Met Arg Ala
                   245                 250                 255
   Ile Thr Glu Ser Gly Leu Arg Val Gly Ala Asp Ile Ser Val Val Gly
               260                 265                 270
   Tyr Asp Asp Thr Glu Asp Ser Ser Cys Tyr Ile Pro Pro Ser Thr Thr
           275                 280                 285
   Ile Lys Gln Asp Phe Arg Leu Leu Gly Gln Thr Ser Val Asp Arg Leu
       290                 295                 300
   Leu Gln Leu Ser Gln Gly Gln Ala Val Lys Gly Asn Gln Leu Leu Pro
   305                 310                 315                 320
   Val Ser Leu Val Lys Arg Lys Thr Thr Leu Ala Pro Asn Thr Gln Thr
                   325                 330                 335
   Ala Ser Pro Arg Ala Leu Ala Asp Ser Leu Met Gln Leu Ala Arg Gln
   340 345 350
   Val Ser Arg Leu Glu Ser Gly Gln
   355 360
(2) ANGABEN ZU SEQ ID NO: 10:
   (i) SEQUENZKENNZEICHEN:
      (A) LÄNGE: 91 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 10:
   Met Val Arg Trp Thr Leu Trp Asp Thr Leu Ala Phe Leu Leu Leu Leu
   1 5 10 15
   Ser Leu Leu Leu Pro Ser Leu Leu Ile Met Phe Ile Pro Ser Thr Phe
   20 25 30
   Lys Arg Pro Val Ser Ser Trp Lys Ala Leu Asn Leu Arg Lys Thr Leu
   35 40 45
   Leu Met Ala Ser Ser Val Arg Leu Lys Pro Leu Asn Cys Ser Arg Leu
   50 55 60
   Pro Cys Val Tyr Ala Gln Glu Thr Leu Thr Phe Leu Leu Thr Gln Lys
   65 70 75 80
   Lys Thr Cys Val Lys Asn Tyr Val Gln Lys Glu
   85 90

## Beschreibung

Die vorliegende Erfindung betrifft ein Verfahren zur Selektion von mutierten O_{R}- oder O_{L}-Operatorsequenzen aus lambdoiden Phagen, die eine im Vergleich zur Wildtypsequenz erhöhte Thermostabilität hinsichtlich der Bindung eines temperatursensitiven Repressors aufweisen. Weiterhin werden neue mutierte O_{R}- oder O_{L}-Operatorsequenzen sowie deren Verwendung zur temperaturregulierten Expression von Genen und zur Herstellung verbesserter Impfstoffe offenbart.

Die Initiation der Transkription von der O_{R}-O_{L}-Region des Bakteriophagen Lambda und anderer lambdoider Phagen wird durch einen Repressor, das Produkt des cl-Gens, negativ und positiv reguliert (siehe den Übersichtsartikel Ptashne et al., Cell 19 (1980), 1-11). In der O_{R}-Region überlappen drei Operatorsequenzen (O_{R}1, O_{R}2 und O_{R}3) die in unterschiedlichen Richtungen orientierten Promotoren P_{R} und P_{RM}. P_{R} steuert die Transkription von Genen, welche für den lytischen Vermehrungszyklus des Phagen verantwortlich sind, während P_{RM} der Promotor für das Lambda cl-Gen ist, welches für das Aufrechterhalten des lysogenen Zustands verantwortlich ist. Der Lambda-Repressor cl bindet kooperativ an die Operatorsequenzen O_{R}1 und O_{R}2 mit dem Ergebniss, daß P_{R} reprimiert und P_{RM} aktiviert wird.

Darüberhinaus enthält der Bakteriophage Lambda auch eine weitere Operatorregion O_{L}, die ebenfalls drei Operatorsequenzen (O_{L}1, O_{L}2 und O_{L}3) enthält. Durch Bindung des cl-Repressors an diese O_{L}-Operatorregion kann die Expression des Lambda N-Gens vom P_{L}-Promotor reprimiert werden.

Promotoren des Bakteriophagen Lambda, insbesondere der P_{L}- und der P_{R}-Promotor werden in der rekombinanten DNA-Technologie seit langem zur heterologen temperaturregulierten Genexpression in E.coli verwendet (vgl. Hedgpeth et al., Molec.Gen.Genet. 183 (1978), 197-203 und Bernard et al., Gene 5 (1979), 59-76; Buell et al., Nucleic Acids Res. 13 (1985), 1923 und Shatzman und Rosenberg, Methods Enzymol. 152 (1987), 661). Bei diesen Expressionssystemen wird ein temperatursensitiver Lambda-Repressor cl857 verwendet, welcher die P_{L}- und P_{R}-Transkription bei geringen Temperaturen bis 30°C reprimiert, aber bei höheren Temperaturen eine Genexpression ermöglicht.

Hershberger et al. (J. Biol. Chem. 268 (1993), 8943-8948) beschreiben Expressionsuntersuchungen am P_{RM}-Promotor des Bakteriophagen Lambda. Dabei wird eine Mutation zur Aktivierung des P_{RM}-Promotors, eine Mutation zur Inaktivierung des P_{R}-Promotors sowie eine Mutation zur vollständigen Inaktivierung des O_{R}2-Operators untersucht.

Ein Vorteil dieses Lambda-Expressionssystems besteht darin, daß die Induzierung der Genexpression auf einfache Weise durch Temperaturerhöhung bewerkstelligt werden kann und daß hierzu keine Zugabe chemischer Induktoren erforderlich ist. Ein schwerwiegender Nachteil ist jedoch, daß die Repression der Genexpression nur bis zu relativ geringen Temperaturen von maximal 30°C erfolgen, einer Temperatur, bei der ein nur langsames Bakterienwachstum stattfindet. Die der Erfindung zugrundeliegende Aufgabe bestand somit darin, ein verbessertes System zur Lambda-P_{L}- oder P_{R}-Genexpression bereitzustellen, welches eine Repression bei variablen höheren Temperaturen ermöglicht.

Diese Aufgabe wird gelöst durch Bereitstellung mutierter O_{R}- oder O_{L}-Operatorsequenzen aus lambdoiden Phagen, die eine im Vergleich zur Wildtypoperatorsequenz höhere Thermostabilität hinsichtlich der Bindung eines temperatursensitiven Repressors aufweisen. Die Erkenntnis, daß überhaupt Lambda-Expressionssysteme mit verbesserter Thermostabilität hergestellt werden können, ist höchst überraschend, da außer der temperatursensitiven Lambda cl857-Mutante keine weiteren temperatursensitiven Repressormolekül kodierenden DNA-Sequenz befinden. So ist beispielsweise aus der Literatur eine Mutation der Lambda-O_{R}2-Operatorsequenz bekannt, welche zu einem völligen Verlust der Repressorbindung führt (Hawley et al., J.Biol.Chem. 260 (1985), 8618-8626).

Zur Identifizierung geeigneter Mutanten wird ein Verfahren bereitgestellt, das die Selektion von mutierten O_{R}- oder O_{L}-Operator-DNA-Sequenzen aus lambdoiden Phagen ermöglicht, die eine im Vergleich zur Wildtypsequenz erhöhte Thermostabilität hinsichtlich der Bindung eines Repressors aufweisen, wobei das Verfahren dadurch gekennzeichnet ist, daß man (a) eine DNA-Kassette herstellt, die ein Selektionsgen unter operativer Kontrolle einer Expressionskontrollsequenz, umfassend mindestens eine O_{R}- oder O_{L}-Operatorsequenz aus einem lambdoiden Phagen und einem Promotor enthält, (b) die Operator-DNA-Sequenz einer Mutagenese unterzieht, (c) eine Selektion der mutierten Operator-DNA-Sequenzen durchführt, die eine im Vergleich zur Wildtyp-Sequenz erhöhte Thermostabilität hinsichtlich der Bindung eines temperatursensitiven Repressors aufweisen und (d) die mutierten Operator-DNA-Sequenzen analysiert.

Die lambdoiden Phagen werden vorzugsweise ausgewählt aus der Gruppe bestehend aus Phage Lambda, Phage 21, Phage 22, Phage 82, Phage 424, Phage 434, Phage D326, Phage DLP12, Phage Gamma, Phage HK022, Phage P4, Phage Phi80, Phage Phi81, Coliphage 186 und rekombinanten Variationen davon. Die genannten Phagen sind hinsichtlich des Mechanismus der Repression der Genexpression über einen cl-Repressor sehr ähnlich (Johnson et al., Nature 294 (1981), 217-223). Rekombinante Variationen der genannten Phagen, z.B. Lambda imm434 können durch Austausch einzelner Genomfragmente innerhalb der genannten Phagen erhalten werden (vgl. hierzu Hendricks et al., Lambda 2 (1983), R.W. Hendricks, J.W.Roberts, F.W. Stahl und R.A.Weissberg (HRSG), Cold Spring Harbor Laboratory Press, New York). Vorzugsweise wird als lambdoider Phage der Phage Lambda oder eine rekombinante Variation davon, z.B. Lambda imm434 verwendet. Besonders bevorzugt wird zur Mutagenese eine Operator-DNA-Sequenz aus den Operatorregionen O_{R} (SEQ ID NO. 1) oder/und O_{L} (SEQ ID NO. 3) des Phagen Lambda und insbesondere eine der darin enthaltenen Operatorsequenzen O_{R}1, O_{R}2 und O_{R}3 bzw. O_{L}1, O_{L}2 und O_{L}3 verwendet. Am meisten bevorzugt ist die Operatorsequenz O_{R}2.

Das Selektionsgen für die DNA-Kassette, welches unter operativer Kontrolle der die mutierte Operatorsequenz enthaltenden Expressionskontrollsequenz, vorzugsweise einer Lambda-Operator/Promotor-Region, gebracht wird, ist vorzugsweise ein Suizidgen, welches bei seiner Expression zum Tod der Bakterienzelle führt und somit als Selektionsmarker zur Identifizierung geeigneter Mutanten dient. Das Suizidgen soll bei einer Temperatur, bei der der Lambda-Repressor an die mutierte Operatorsequenz bindet, so stark reprimiert werden, daß eine die DNA-Kassette enthaltende Bakterienzelle wachsen kann. Bei Überschreiten der maximalen Temperatur, bei der der Repressor noch an den Operator bindet, erfolgt eine Expression des Suizidgens und eine Zerstörung der Bakterienzelle. Auf diese Weise gelingt eine einfache und direkte Selektion von geeigneten mutierten Operatorsequenzen. Ein geeignetes Suizidgen ist das E-Lysegen aus dem Phagen PhiX174 sowie Homologe und davon abgeleitete Derivate (Hutchison und Sinsheimer, J.Mol.Biol. 18 (1966), 429-447; Witte et al., Multifunctional safety vector systems for DNA cloning, controlled expression of fusion genes, and simplified preparation of vector DNA and recombinant gene products, in BioTech Forum, Advances in Molecular Genetics 3, pp 219-239, Hrsg: Issinger, O.-G., Henke, J., Kämpf, J., Driesel, A.J., Hüthing Verlag 1991, Heidelberg). Weitere Beispiele für geeignete Lysegene sind GEF (Poulsen et al., Mol.Microbiol. 5 (1991), 1627-1637) und Kil (Reisinger et al., Virology 193 (1993), 1033-1036). Andererseits kann das Selektionsgen auch ein Reportergen, wie z.B. das β-Gal-Gen sein.

Die Operator-DNA-Sequenz wird zur Herstellung von Mutanten vorzugsweise einer ortsspezifischen Mutagenese mittels eines oder mehrerer Oligonucleotide beispielsweise nach der Methode von Kunkel (Proc.Natl.Acad.Sci. USA 82 (1985), 488-492) unterzogen oder durch Selektion in einem Mutator-Bakterienstamm, z.B. einem E.coli mutD oder mutL Mutatorstamm wie etwa E.coli ES1578 (Wu et al., Gene 87 (1990), 1-5) erhalten. Die Selektion der mutierten Operator-DNA-Sequenzen erfolgt vorzugweise durch Bestimmung der Bindefähigkeit mit einem temperatursensitiven cl-Repressor, insbesondere dem temperatursensitiven cl857-Repressor. Hierzu wird die DNA-Kassette, die sich vorzugsweise auf einem Vektor befindet, in eine Bakterienzelle transformiert, die ein für einen temperatursensitiven cl-Repressor kodierendes Gen enthält. Dieses Gen kann ebenfalls auf einem Vektor vorliegen (Remaut et al., Gene 15 (1981), 81-93). Andererseits kann eine Bakterienzelle verwendet werden, die ein solches Repressorgen in seinem Chromosom enthält, z.B. E.coli M5219 (vgl. z.B. Shimatake und Rosenberg, Nature 292 (1981), 128).

Durch Kultivierung der mit einer Lysekassette transformierten Bakterienzellen, die mutierte Operator-DNA-Sequenzen enthalten, können auf einfache Weise Mutanten identifiziert werden, die bei unterschiedlich hohen Temperaturen gegenüber einer Lyse resistent sind. Bisher konnten mehrere Mutanten identifiziert werden, die bei Temperaturen bis 33°C, 35°C, 37°C und 39°C gegenüber einer Lyse resistent sind. Diese Bakterien enthalten mutierte Operator-DNA-Sequenzen, die eine Bindung des Repressors bis zu der jeweils angegebenen Temperatur ermöglichen. Ein besonders bevorzugtes Beispiel ist eine Mutante, an die der cl857-Repressor bis zu einer Temperatur von etwa 37°C bindet. Die Mutation gegenüber dem Wildtyp ist ein einziger Basenaustausch im O_{R}2-Abschnitt der Lambda-O_{R}-Operatorregion. Die Sequenz dieses mutierten Lambda-O_{R}-Operators ist in SEQ ID NO. 2 gezeigt.

Es werden weiterhin mutierte O_{R}- oder O_{L}-Operatorsequenzen aus lambdoiden Phagen, die eine im Vergleich zur Wildtyp-Sequenz erhöhte Thermostabilität hinsichtlich der Bindung eines Repressors aufweisen, und die durch das oben beschriebene Selektionsverfahren erhältlich sind, beschrieben. Die mutierten O_{R}- oder O_{L}-Operatorsequenzen besitzen eine erhöhte Thermostabilität hinsichtlich der Bindung eines temperatursensitiven Repressors, insbesondere des temperatursensitiven cl-Repressors. Die mutierten Operatorsequenzen weisen eine um etwa 3 bis 10°C, insbesondere eine um etwa 7 bis 9°C erhöhte Thermostabilität gegenüber der Wildtyp-Sequenz auf.

Da das erfindungsgemäße Selektionsverfahren vorzugsweise an O_{R}- oder O_{L}-Operatorsequenzen durchgeführt wird, die aus dem Phagen Lambda stammen, betrifft die vorliegende Erfindung insbesondere mutierte Lambda O_{R}- oder O_{L}-Operatorsequenzen, die Varianten der in SEQ ID NO. 1 gezeigten O_{R}-Operatorsequenzen oder Varianten der in SEQ ID NO. 3 gezeigten O_{L}-Operatorsequenzen sind. Unter Variante ist in diesem Zusammenhang eine Operatorsequenz zu verstehen, die sich von der Wildtypsequenz in mindestens einer Sequenzposition durch Insertion, Deletion oder Austausch von Basen unterscheidet. Besonders bevorzugt sind die Unterschiede im Bereich der Abschnitte O_{R}1, O_{R}2 und O_{R}3 bzw. O_{L}1, O_{L}2 und O_{L}3. Ein spezifisches Beispiel für eine erfindungsgemäße mutierte Lambda-Operatorsequenz ist die in SEQ ID NO. 2 gezeigte Lambda-O_{R}-Operatorsequenz.

Die mutierten Operatorsequenzen erlauben die Herstellung von neuen temperaturregulierten Systemen zur Genexpression, bei denen die Kultivierung von Mikroorganismen, insbesondere Bakterien, im reprimierten Zustand bei variablen Temperaturen, vorzugsweise bei höheren Temperaturen als bisher, insbesondere 33 bis 39°C erfolgen kann. Ein Gegenstand der vorliegenden Erfindung ist somit ein Verfahren, bei dem die mutierten O_{R}- oder O_{L}-Operatorsequenzen zur temperaturregulierten Expression von Genen in Bakterien, insbesondere in gram-negativen Bakterien wie etwa E.coli verwendet werden. Durch Kombination einer Wildtyp-O_{R}- oder O_{L}-Operatorregion und mindestens einer Operatorregion, die eine erfindungsgemäße mutierte Operatorsequenz enthält, oder durch Kombination mehrerer Operatorregionen, die mutierte erfindungsgemäße Operatorsequenzen mit erhöhter Thermostabilität enthalten, kann sogar eine temperaturregulierte sequentielle Expression von Genen erreicht werden.

Vektoren und Bakterienstämme, in denen die erfindungsgemäßen mutierten Operatorsequenzen zur temperaturregulierten Expression von Genen eingesetzt werden können, sind dem Fachmann geläufig. Hier kann auf die aus dem Stand der Technik bekannten Expressionssysteme mit dem Lambda cI857-Repressor in Kombination mit einem geeigneten Promotor, z.B. dem Lambda-P_{L} oder dem Lambda-P_{R}-Promotor zurückgegriffen werden (vgl. z.B. Sambrook et al., Molecular Cloning, A Laboratory Manual, 2nd Edition, 1989, Cold Spring Harbor Laboratory Press, New York, 17.11-17.12).

Ein weiterer Gegenstand der vorliegenden Erfindung ist eine Nucleinsäure, umfassend eine bakterielle Expressionskontrollsequenz, d.h. eine Promotor- und Operatorregionen enthaltende Sequenz, die eine erfindungsgemäße mutierte O_{R}- oder O_{L}-Operatorsequenz enthält, in operativer Verknüpfung mit einer Protein-kodierenden Sequenz. Die Protein-kodierende Sequenz kann beispielsweise eine für ein eukaryontisches Protein oder Polypeptid kodierende Sequenz oder aber auch ein bakterielles Gen, z.B. das E-Lysegen sein.

Ein weiterer Gegenstand der vorliegenden Erfindung ist ein Vektor, der mindestens eine Kopie der bakteriellen Expressionskontrollsequenz in operativer Verknüpfung mit der Protein-kodierenden Sequenz enthält. Dieser Vektor kann ein beliebiger prokaryontischer Vektor sein, z.B. ein chromosomaler Vektor wie etwa ein Bakteriophage oder ein extrachromosomaler Vektor wie etwa ein Plasmid. Geeignete prokaryontische Vektoren sind z.B. bei Sambrook et al., Supra, Kapitel 1-4, beschrieben.

Noch ein weiterer Gegenstand der vorliegenden Erfindung ist eine Bakterienzelle, die mit einer erfindungsgemäßen Nucleinsäure oder einem erfindungsgemäßen Vektor transformiert ist. In einer bevorzugten Ausführungsform ist die Zelle eine gram-negative prokaryontische Zelle, besonders bevorzugt eine E.coli-Zelle. Vorzugsweise enthält die Zelle die Nucleinsäure oder den Vektor in ihrem Chromosom integriert und enthält weiterhin ein Gen für einen cI-Repressor aus einem lambdoiden Phagen, insbesondere das Gen für den Lambda-cI857-Repressor.

Eine besonders bevorzugte Anwendung der erfindungsgemäßen mutierten Operatoren liegt auf dem Gebiet der Impfstoffherstellung. Aus dem Stand der Technik sind sogenannte "Bakterienghosts" als Impfstoffe bekannt, d.h. Bakterienhüllen, die mittels Protein-E-induzierter Lyse aus gram-negativen Bakterien, z.B. E.coli Salmonella typhimurium, Klebsiella pneumoniae, Actinobacillus pleuropneumoniae etc. hergestellt werden konnten. Diese Ghosts, die in der Beschaffenheit ihrer Zelloberfläche sowie den vom Immunsystem erkennbaren Repertoire an Oberflächenantigenen dem aktiven Pathogen weitgehend gleichen, rufen in verschiedenen Tiermodellen eine protektive zelluläre oder/und humorale Immunantwort hervor.

Die Herstellungsweise der Ghosts beruht auf der stringent kontrollierten Expression des E-Lysegens aus PhiX174, dessen Expressionsprodukt einen Tunnel durch die bakterielle Zellwandhülle ausbildet und so zum Ausströmen des Zellinhalts der Wirtszelle führt. Die Regulation dieses für die Zelle letalen Gens kann über einen Lambda-Repressor ausgeübt werden, z.B. den temperatursensitiven Lambda-Repressor cI857, der wie zuvor ausgeführt bei Temperaturen über 30°C seine Funktion verliert. Dies bedingte, daß die bisher zur Produktion von Bakterienghosts verwendeten Bakterienkulturen bei niedrigen Temperaturen, bevorzugt bei 28°C, angezogen werden mußten.

Gleichwohl es mit dieser Methodik zu befriedigenden Resultaten bezüglich der Immunogenität der hergestellten Ghosts gekommen ist, ist eine Verbesserung der Bakterienkultivierung dringend erstrebenswert, da das Repertoire der antigenen Determinanten auf der bakteriellen Oberfläche sich in Abhängigkeit der äußeren Bedingungen ändern kann. Da pathogene Bakterien, die Mensch oder Tier befallen, meist in einer Umgebungstemperatur von 37 bis 39°C siedeln, sollte diese "natürliche" Umgebungstemperatur auch während des Herstellungsverfahrens von Ghosts eingehalten werden können.

Ein Verfahren zur Herstellung von Bakterienghosts, welches diese Aufgabe löst, wird durch Verwendung der erfindungsgemäßen mutierten Operatorsequenzen bereitgestellt. Diese Operatorsequenzen erlauben bis zu einem Temperaturbereich von vorzugsweise 35 bis 39°C das Wachstum der Bakterien und erlauben bei einer Temperaturerhöhung von 37 bis 42°C die Lyse. Dieses veränderte Lyseverhalten ermöglicht die Anzucht der Krankheitserreger nahe der Körpertemperatur des Impfkandidaten, was für die Zusammensetzung der äußeren Membran äußerst wichtig ist. Darüberhinaus kann die neue Lysekassette auch als Sicherheitskassette bei Lebendvakzinen eingesetzt werden, da z.B. im Menschen bei Induktion von Fieber (39°C) die Abtötung der Impfbakterien erfolgt.

Ein Gegenstand der vorliegenden Erfindung ist somit eine Impfstoffzusammensetzung, die eine lebende erfindungsgemäße Bakterienzelle als Wirkstoff gegebenenfalls mit pharmazeutisch verträglichen Hilfs-, Zusatz- und Trägerstoffen enthält. Die lebende Bakterienzelle enthält eine Nucleinsäure, umfassend eine bakterielle Expressionskontrollsequenz mit einer mutierten Operatorsequenz in operativer Verknüpfung vorzugsweise mit einem Lysegen. Noch ein weiterer Gegenstand der vorliegenden Erfindung ist ein Impfstoffzusammensetzung, die einen Bakterienghost als Wirkstoff gegebenenfalls mit pharmazeutisch verträglichen Hilf-, Zusatz- und Trägerstoffen enthält, wobei der Bakterienghost durch Kultivierung einer erfindungsgemäßen Bakterienzelle bei Temperaturen von 35 - 39°C und anschließende Lyse der Bakterienzelle durch Temperaturerhöhung erhältlich ist. Als Impfstoffe geeignete Bakterienzellen sind insbesondere gram-negative Bakterien wie etwa E.coli, beispielsweise die Stämme STEC, EHEC, O78:K80, Salmonellen wie etwa S.choleraesuis, S.enteritidis und S.typhimurium, Pasteurella multocida, Pasteurella haemolytica, Bordetella bronchiseptica, Klebsiella pneumoniae, Actinobacillus pleuropneumoniae, Haemophilus influenzae, Vibrio cholerae, Helicobacter pylori, Alcaligenes eutrophus, Campylobacter jejuni und Pseudomonas aeruginosa.

Die erfindungsgemäßen modifizierten Impfstoffzusammensetzungen können oral, aerogen oder parenteral auf die Impfkandidaten übertragen werden. Dabei wird bei der Impfstoffapplikation vorzugsweise der natürliche Weg gewählt, den entsprechenden Mikroorganismen für die Infektion und die Anfangsstadien der Etablierung einer Infektionskrankheit wählen. Da bei den erfindungsgemäßen Vakzinen alle Oberflächeneigenschaften erhalten bleiben, kann durch diese Applikation eine lokale Induktion der Immunantwort erfolgen, wie sie auch beim natürlichen Infektionsprozess auftritt.

Wie oben ausgeführt, können durch Anwendung erfindungsgemäßer mutierter Operatorsequenzen Vakzine entwickelt werden, die bei Überschreiten einer Sollwert-Temperatur kontrolliert lysiert werden. Weiterhin kann jedoch auch eine kältesensitive Suizidkassette bereitgestellt werden, die gram-negative Bakterien, die als Lebendvakzine eingesetzt werden, bei Freisetzung in die Umwelt abtötet. So kann durch Kombination von zwei genetischen Regulationssystemen ein Absterben der Bakterien bei Unterschreiten eines Sollwerts der Umgebungstemperatur durch Expression des Suizidgens erfolgen. Diese Sicherheitskassette gewährleistet die Abtötung von Lebendvakzinen auch bei einer Ausscheidung aus dem Organismus.

Die Erfindung betrifft somit eine Nucleinsäure, umfassend (a) eine erste bakterielle Expressionskontrollsequenz, die eine erfindungsgemäße mutierte O_{R}- oder O_{L}-Operatorsequenz aus einem lambdoiden Phagen enthält und an die ein erster temperatursensitiver cI Repressor aus lambdoiden Phagen binden kann, in operativer Verknüpfung mit einer für einen zweiten Repressor kodierenden Sequenz, wobei der zweite Repressor nicht an die erste bakterielle Expressionssequenz binden kann, und (b) eine zweite bakterielle Expressionskontrollsequenz, an die der zweite Repressor binden kann, in operativer Verknüpfung mit einem Suizidgen.

Die Komponenten (a) und (b) können kovalent miteinander verknüpft, z.B. auf einen einzigen Vektor, vorliegen oder auch voneinander getrennt, z.B. auf unterschiedlichen Vektoren, sein, oder getrennt oder gemeinsam auf dem Chromosom eines Empfängerbakteriums lokalisiert sein.

Noch ein weiterer Gegenstand der vorliegenden Erfindung ist eine Bakterienzelle, die mindestens eine Kopie einer wie zuvor definierten Nucleinsäure enthält. Weiterhin enthält die Bakterienzelle zweckmäßigerweise ein Gen für den ersten Repressor. Der erste Repressor ist vorzugsweise der temperatursensitive cI857-Repressor.

Die erfindungsgemäße Sicherheitskassette enthält vorzugsweise ein Gen, welches für einen temperatursensitiven cI-Repressor, z.B. den Repressor cI857 kodiert, und ein Gen, das für einen zweiten Repressor kodiert, wobei dieses Gen unter Kontrolle einer Lambda-Promotor/Operator-Region steht, an die der temperatursensitive Repressor bindet. Der zweite Repressor steuert wiederum die Expression eines anderen Gens, z.B. eines Suizidgens, wie das E-Lysegen. Bei 37°C ist der temperatursensitive Lambda-Repressor inaktiv, so daß der zweite Repressor exprimiert wird, wodurch wiederum die Expression des Suizidgens reprimiert wird.

Bei Verringerung der Temperatur bindet der temperatursensitive Lambda-Repressor an den Operator, so daß die Expression des zweiten Repressors blockiert wird, was zu einer Expression des Suizidgens führt. Bevorzugt ist für diese Sicherheitskassette eine erste Expressionskontrollsequenz, die den mutierten Lambda-Operator enthält, da hierbei eine bessere und schnellere Aktivierung des Suizidgens erhalten wird.

Der zweite Repressor kann ein beliebiger Repressor sein, ein lac-Repressor. Bevorzugt ist jedoch die Verwendung eines weiteren Repressors aus lambdoiden Phagen, z.B. cI aus dem Phagen 434, der nicht temperatursensitiv ist und an eine eigene Operatorsequenz, aber nicht an die vom Lambda-Repressor cI857 erkannte Sequenz bindet.

Besonders bevorzugt ist es, für die Entwicklung von Lebendimpfstoffen sowohl eine Hitze- als auch ein Kälteregulationselement einzubauen. Dieses Einbauen erfolgt vorzugsweise durch homologe Rekombination in das Chromosom des Impfbakteriums.

Somit betrifft die vorliegende Erfindung auch eine Bakterienzelle, die neben den beiden Komponenten (a) und (b) als Komponente (c) eine dritte bakterielle Expressionskontrollsequenz, die eine erfindungsgemäße mutierte Operatorsequenz enthält, in operativer Verknüpfung mit einem Suizidgen umfaßt.

Auch diese Bakterienzellen können in Impfstoffzusammensetzungen insbesondere für Lebendvakzine eingesetzt werden. Auf diese Weise können wärme- oder/und kälteempfindliche Sicherheitslebendvakzine bereitgestellt werden, die bei einer Erhöhung der Körpertemperatur des Impfkandidaten, z.B. durch Fieber, oder/und bei Ausscheidung in die Umgebung zu einem Absterben der Impfbakterien führen.

Weiterhin soll die Erfindung durch die nachfolgenden Figuren, Sequenzprotokolle und Beispiele erläutert werden.

Es zeigen:
- Fig. 1a: die schematische Darstellung einer Lysekassette des Standes der Technik, umfassend eine Lambda-O_{R}-Wild- typ-Region, das Lambda-cI857-Gen unter Kontrolle des Promotors P_{RM} und das E-Lysegen unter Kontrolle des Promotors P_{R};
- Fig. 1b: die schematische Darstellung einer erfindungsgemäßen Lysekassette, die eine mutierte Lambda-O_{R}-Sequenz enthält;
- Fig. 2a: die schematische Darstellung einer kälteempfindli- chen Sicherheitskassette, umfassend eine Wildtyp (pCS1) bzw. mutierte (pCSJ1) O_{R}-Operatorsequenz, das Lambda-cI857-Gen unter Kontrolle des Promotors P_{RM}, das Gen des lacI-Repressors unter Kontrolle von P_{R} und das E-Lysegen unter Kontrolle des lac-Promotor/- Operatorsystems, bei einer Temperatur, bei der der temperatursensitive Lambda-Repressor cI857 nicht an die Lambda O_{R}-Sequenz bindet;
- Fig. 2b: die schematische Darstellung der Sicherheitskassette gemäß Fig. 2a bei einer Temperatur, bei der der Lambda-Repressor cI857 an den Lambda O_{R}-Operator bindet;
- Fig. 3: die Lysekurve von Bakterienzellen (optische Dichte gegen Zeit), die ein Plasmid mit der in Fig. 1b ge- zeigten Lysekassette enthalten;
- Fig. 4: die Lysekurve einer Bakterienzelle, die eine kälte- sensitive Sicherheitskassette mit dem Wildtyp O_{R}- Operator enthält und
- Fig. 5: ein Vergleich der Lysekurven von Bakterienzellen, die eine kältesensitive Sicherheitslysekassette mit dem Wildtyp O_{R}-Operator (pCS1) bzw. dem mutierten Operator (pCSJ1) enthalten,
- Fig. 6a: die schematische Darstellung einer kältesensitiven Sicherheitskassette, umfassend eine Wildtyp (pCS2) bzw. mutierte (pCSJ2) O_{R}-Operatorsequenz, das Lambda- cI857-Gen unter Kontrolle des Promotors P_{RM}, das Gen des Phage 434 cI-Repressors unter Kontrolle von Lambda P_{R} und das E-Lysegen unter Kontrolle des 434 O_{R} (P_{RM}-P_{R}) Promotor/Operator-Systems, bei einer Tem- peratur, bei der der temperatursensitive Lambda-Re- pressor cI857 nicht an die Lambda-O_{R}-Sequenz bindet,
- Fig. 6b: die schematische Darstellung der Sicherheitskassette gemäß Fig. 6a bei einer Temperatur, bei der der Lambda-Repressor cI857 an den Lambda O_{R}-Operator bindet;

- SEQ ID NO. 1: die Nucleotidsequenz des Lambda-O_{R}-Operators; die Operatorsequenz O_{R}3 reicht von Position 11 - 27; die Operatorsequenz O_{R}2 reicht von Posi- tion 34 - 41; die Operatorsequenz O_{R}1 reicht von Position 58 - 74;
- SEQ ID NO. 2: die Nucleotidsequenz eines mutierten Lambda-O_{R}- Operators, die gegenüber der Wildtypsequenz einen Austausch von T → C an Position 42 auf- weist;
- SEQ ID NO. 3: die Nucleotidsequenz des Lambda-O_{L}-Operators; die Operatorsequenz O_{L}3 reicht von Position 11 - 27; die Operatorsequenz O_{L}2 reicht von Posit- ion 31 - 47; die Operatorsequenz O_{L}1 reicht von Position 55 - 70;
- SEQ ID NO. 4 bis 6: ein 1601 bp langes DNA-Fragment des Plasmids pAW12; bp 1 - 983 stammen aus dem Bakteriopha- gen Lambda (Position 37125 - 38107; vgl. Sanger et al., J.Mol.Biol. 162 (1982), 729-773) und enthalten das Lambda-cI857-Gen (Position 816- 106; SEQ ID NO. 5) sowie die mutierte O_{R}-Opera- torregion (Mutation an Position 858 T → C); bp 1023 - 1601 stammen aus dem Phagen PhiX174 (Po- sition 447 - 1026; vgl. Sanger et al., J.Mol.Biol. 125 (1978), 225-246) und enthalten das E-Lysegen (Position 1144-1416; SEQ ID NO. 6);
- SEQ ID NO. 7 bis 10: ein 2834 bp langes DNA-Fragment des Plasmids pCSJ; bp 1 - 983 stammen aus dem Bakteriophagen Lambda (Position 37125 - 38107) und enthalten das cI857-Gen (Postion 816-106; SEQ ID NO. 5) sowie die mutierte Lambda-O_{R}-Region (Mutation an Position 858 T → C); bp 990 - 2230 stammen aus dem E.coli lac-Operon subkloniert auf dem Plasmid pMC7 (Calos, Nature 274 (1978), 762- 765) und enthalten das lacI-Repressorgen (bp 1025-2104; SEQ ID NO. 9) und den lac-Promotor/- Operator; bp 2256-2834 stammen aus dem Bakte- riophagen PhiX174 (Position 447 - 1026) und enthalten das E-Lysegen (bp 2377-2649; SEQ ID NO. 10).

### BEISPIELE

### Beispiel 1:

### 1.1. Random-Mutagenese der Lambda-O_{R}-Operatorregion

Als Ausgangsmaterial wurde das Plasmid pAW12 (Witte und Lubitz, Eur.J.Biochem. 180 (1989), 393-398) gewählt, welches das Lysegen E aus dem Bakteriophagen PhiX174 unter Kontrolle des Lambda-P_{R}-Promotors sowie das zugehörige Repressorgen cI857 enthält. Ziel dieses Versuchs war eine Veränderung der Lysekassette, so daß das Lysegen E nicht bereits bei 30°C, sondern bei höheren Temperaturen aktiviert wird. Hierzu wurde der E.coli Mutatorstamm ES1578 (Wu et al., (1990), supra) mit dem Lyseplasmid transformiert und eine Selektion auf Klone mit einem veränderten Temperaturprofil der Zellyse durchgeführt.

Dabei wurden die aus der Transformation hervorgegangenen mutierten Klone nach Überstempeln auf Testplatten mit Lyseselektivmedium (LB mit 1% SDS) und Inkubation bei unterschiedlichen Temperaturen (z.B. 33°C, 34°C, 35°C, 36°C, 37°C, 38°C, 39°C, 40°C, 41°C) erkannt. Durch Plasmidextraktion und anschließende Transformation in einen Nicht-Mutatorteststamm wurde das veränderte Lyseprofil der Lysekassette in Flüssigkultur genau bestimmt.

Die Art der Mutation wurde durch Subklonierung der mutagenisierten Lysekassetten in ein Sequenzierplasmid bestimmt. Zusätzlich wurde zur funktionellen Prüfung das Lysegen E gegen das ß-Galactosidasegen ausgetauscht. Anhand eines einfachen β-Gal-Tests konnte dann quantitativ der reprimierte oder aktive Zustand der Genkassette gemessen werden.

Auf diese Weise konnten mehrere Klone mit einem unterschiedlichen Temperaturlyseprofil erhalten werden. Diese Klone erlaubten in einem Temperaturbereich von 33-39°C das Wachstum der Bakterien und führten erst bei einer weiteren Temperaturerhöhung auf 37-42°C zur Lyse der Bakterien.

Durch Sequenzierung eines mutierten Klons, der eine Temperaturbeständigkeit bis 37°C aufweist, wurde eine Mutation der O_{R}-Operatorregion (SEQ ID NO. 2) identifiziert.

### 1.2. Verifizierung der Mutation

Zur Verifizierung der in Beispiel 1.1. erhaltenen Mutation wurde eine ortsspezifische Mutagenese der Lambda O_{R}-Wildtypsequenz unter Verwendung eines Oligonucleotids durchgeführt.

Die Mutagenese wurde ausgeführt nach dem Protokoll von Kunkel (Proc.Natl.Acad.Sci. USA 82 (1985), 488-492).

4 ml Übernachtkultur des E.coli-Stammes CJ236 (dut⁻, ung⁻) wurden in 50 ml LB-Medium (+ 10 µg/ml Chloramphenicol und 0,25 µg/ml Uridin) gegeben und 30 min bei 37°C geschüttelt. Dann wurden 100 µl M13-Phagen zugesetzt und 6 h bei 37°C inkubiert.

Die Kultur wurde in 2 SS34-Zentrifugenröhrchen 10 min bei 14000 Upm und 4°C zentrifugiert, der Überstand in neue Röhrchen dekantiert und zur weiteren Reingigung nochmals zentrifugiert.

Durch Zugabe von 5 ml 5x Polyethylenglycol/NaCl wurden die Phagen 1 h bei 4°C gefällt. Dann wurde 10 min bei 14000 Upm und 4°C zentrifugiert und der Überstand verworfen.

Das Pellet wurde getrocknet, in 0,8 ml TES-Puffer (0,1 M Tris HCl, pH 8; 0,3 M NaCl; 1mM EDTA) suspendiert und 1 h bei 4°C inkubiert. Die Suspension wurde auf 2 Eppendorfgefäße aufgeteilt und 5 min bei 5000 Upm zentrifugiert. Der Überstand, in dem sich die aufgebrochenen Phagen befanden, wurde abgenommen und einer Phenol/Chloroform-Extraktion zur Gewinnung der DNA unterzogen. Die resultierende DNA wurde mit dem 2,5-fachen Volumen 96% Ethanol gefällt, mit 70% Ethanol gewaschen und in 60 µl H₂O aufgenommen.

Ein Oligonucleotid mit der Sequenz 5'-GTA AAA TAG TCA ACA CGC GCG GTG TTA GAT ATT TAT C-3' wurde phosphoryliert. Hierzu wurden 20 µl H₂O, 20 µl Oligonucleotid (20 ng), 4,5 µl Kinase-Puffer (Stratagene) und 0,5 µl Polynucleotidkinase (5 U, Stratagene) 1 h bei 37°C inkubiert. Dann wurden 7 µl 0,1 M EDTA zugegeben und 10 min auf 65°C erhitzt.

Zum Annealing wurden 20 µl phosphoryliertes Oligonucleotid, 3,5 µl einzelsträngige DNA-Matrize (1 µg ssDNA wie zuvor beschrieben hergestellt) und 1,4 µl 20 x SSC-Puffer 5 min auf 70°C erhitzt, langsam bis 25°C abgekühlt und dann auf Eis gestellt.

Zur Extension wurden 10 µl des Reaktionsgemisches aus dem Annealingansatz, 37,5 µl XL-Puffer (27 mM Hepes pH 7,8, jeweils 5 mM dNTP, 13 mM MgCl₂, 2,7 mM Dithiothreitol, 1,3 mM ATP, 1 µl Ligase (1 U, Boehringer Mannheim), 1,5 µl T4-Polymerase (1,5 U, Boehringer Mannheim), 1,5 µl T4-Gen32-Protein (8 µg, Boehringer Mannheim) 10 min auf Eis, 10 min bei Raumtemperatur und 2 h bei 37°C inkubiert. Nach 1 h erfolgte die Zugabe von 1 µl Ligase und 1 µl T4-DNA-Polymerase. Nach Beendigung der Inkubation wurde die Reaktion durch Zugabe von 3 µl 0,25 M EDTA gestoppt.

Zur Transformation wurden 100 µl kompetente E.coli Zellen JM103 (Messing et al., Nucleic Acids.Res. 9 (1981), 309-321) mit 10 µl DNA aus dem Extensionsansatz versetzt und 1 h oder mehr auf Eis inkubiert. Nach einem Hitzeschock für 2,5 min bei 42°C wurden 0,2 ml frische JM103-Zellen in der logarithmischen Wachstumsphase zugesetzt. Die Zellen wurden mit 3 ml Soft Agar vermischt und auf eine LB-Agarplatte ausgeimpft. Anschließend erfolgte Inkubation über Nacht bei 37°C.

Zur Identifizierung der Mutanten wurden mit einer Pasteurpipette Plaques ausgestochen und in 5 ml LB-Medium, dem 400 µl einer Übernachtkultur von E.coli JM103 zugesetzt wurden, angeimpft. Nach 3 h Wachstum bei 37°C wurden die Zellen abzentrifugiert. Aus dem Zellpellet wurden mittels Plasmidpräparation doppelsträngige M13-Plasmide gewonnen. Aus dem Überstand können einzelsträngige M13-Phagen isoliert werden.

### Beispiel 2:

### Analyse der mutagenisierten Lysekassetten

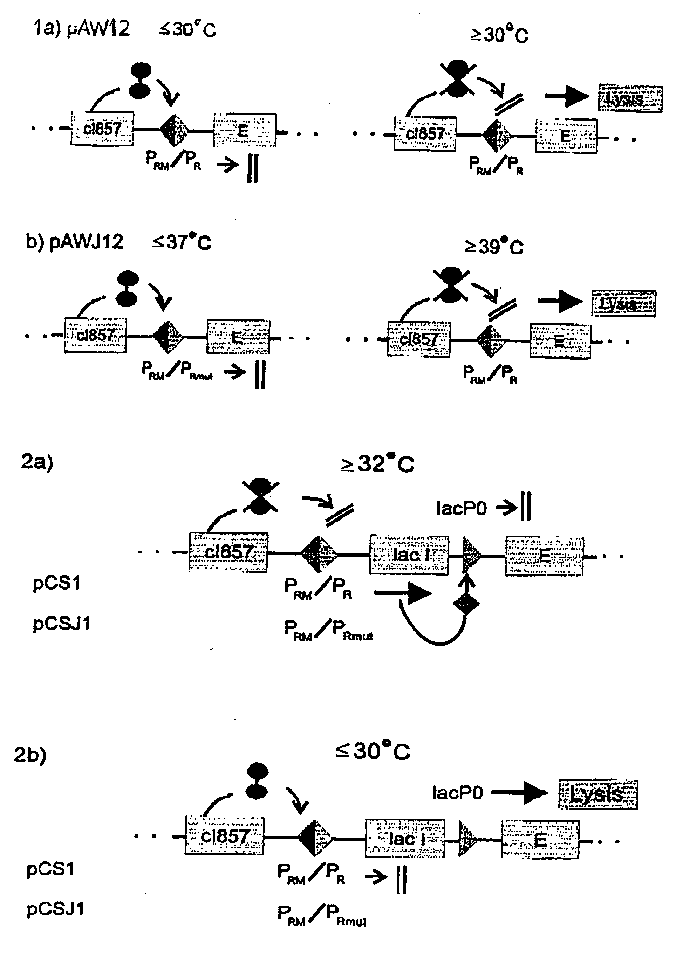

Die Figuren 1 und 2 zeigen unterschiedliche E-spezifische Lysekassetten mit verschiedenen Temperaturinduktionen der Lysefunktion.

In Fig. 1a, welches die Wildtyp-Lambda-O_{R}-Operatorsequenz enthält, erfolgt bis 30°C eine Repression der Funktion des E-Lysegens durch das cI857-kodierte Repressorprotein am vorgeschalteten Lambda-P_{R}-Promotor/Operatorbereich. Bei Temperaturen über 30°C werden cI857-spezifische Repressormoleküle thermisch inaktiviert und die Expression des E-Gens induziert. Fig. 1b zeigt das Plasmid pAWJ12, welches eine mutierte Operatorsequenz (SEQ ID NO. 2) enthält, so daß die Repression der Lysefunktion des Gens E mit Hilfe von cI857 bis 37°C erfolgt und eine Induktion der Lysefunktion erst bei 39°C oder höher erfolgt.

In Fig. 2 ist die Funktion einer kältesensitiven Sicherheitskassette erläutert. Fig. 2a zeigt, daß in den Plasmiden pCS1 (Wildtypoperator) und pCSJ1 (mutierter Operator) bei einer Temperatur von ≥ 32°C (pCS1) bzw. ≥ 39°C (pCSJ1) die Bildung von lacI-spezifischen Repressormolekülen induziert wird, die wiederum die Expression des E-Gens reprimieren. Bei einer Temperatur unterhalb von 30°C (pCS1) bzw. 37°C (pCSJ1) kommt es zur Ausbildung von funktionsfähiger cI857-Repressormoleküle, die die Bildung von lacI-spezifischen Repressormolekülen unterbinden und so die Expression des E-Gens freigeben (Fig. 2b). Im Plasmid pCSJ1 erfolgt die einhergehende Zellyse schneller als in pCS1.

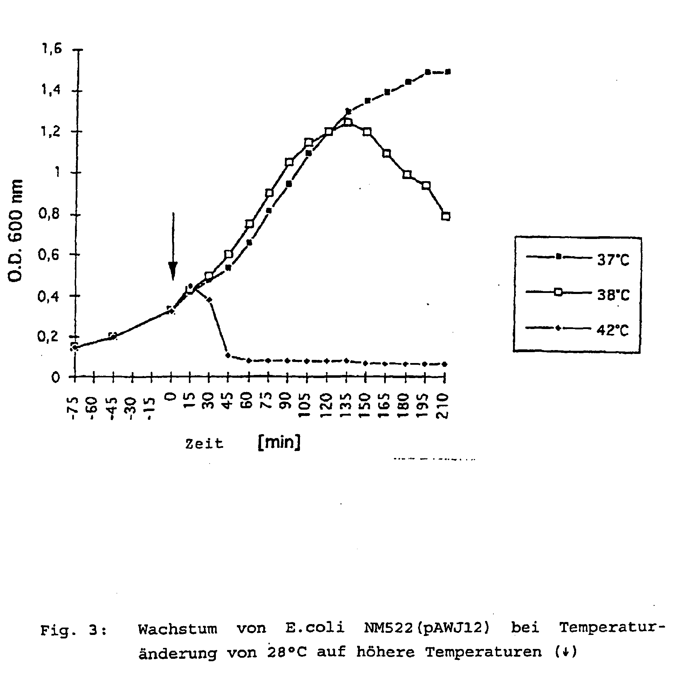

Fig. 3 zeigt die Lysekurve einer das Plasmid pAWJ12 (mutierter Operator) enthaltenden Bakterienzelle. 3 Stunden nach Beginn der Kultivierung wurde die Temperatur von 37°C in einem Aliquot der Bakterienzellen beibehalten, und in zwei anderen Aliquots auf 38 bzw. 42°C erhöht. Bei 37°C findet man ein weiteres Wachstum der Bakterien, während bei 38°C bereits eine Lyse stattfindet. Bei 42°C ist die Lyse deutlich verstärkt.

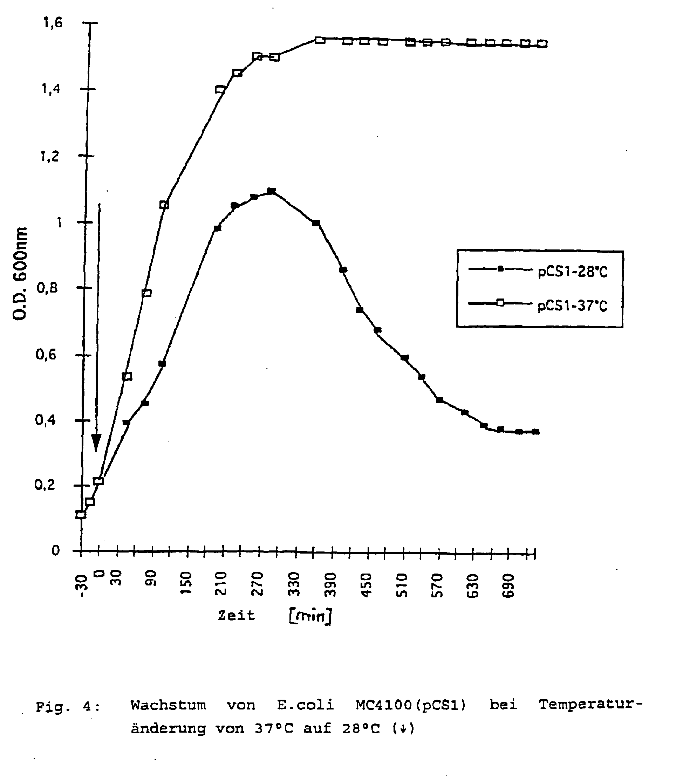

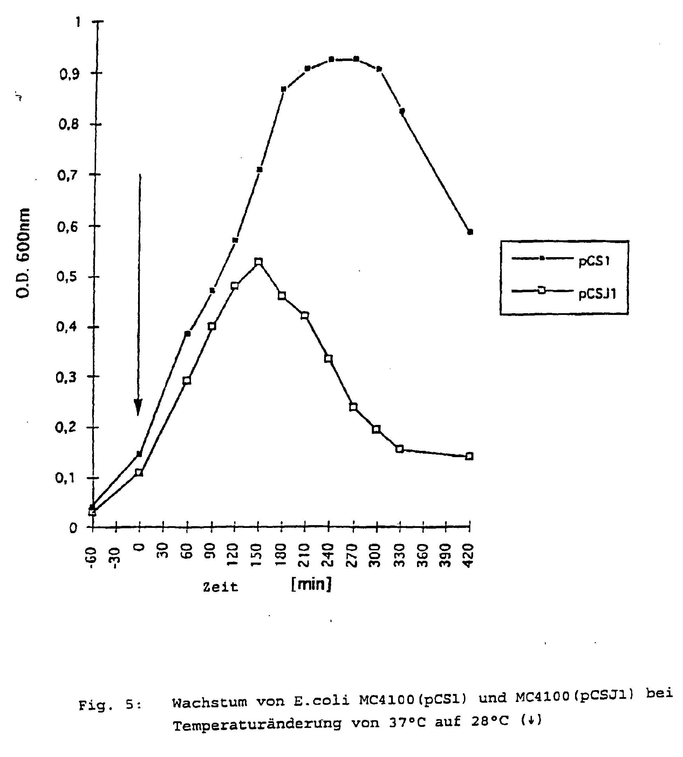

Die Figuren 4 und 5 zeigen die Funktion einer kältesensitiven Sicherheitskassette. In Fig. 4 wurden Bakterienzellen, die das Plasmid pCS1 (Wildtypoperator) enthielten, einer Temperaturänderung von 37 auf 28°C ausgesetzt. Diese Temperaturverringerung führte zu einem Anschalten des E-Lysegens und zu einem Absterben der Zellen (Abnahme der optischen Dichte).

Fig. 5 zeigt einen Vergleich der Lysegeschwindigkeit von Bakterien, die das Plasmid pCS1 (Wildtypoperator) und das Plasmid pCSJ1 (mutierter Operator) enthalten. Es ist zu erkennen, daß die Lyse bei den Bakterien, welche den mutierten Operator enthalten, wesentlich schneller stattfindet.

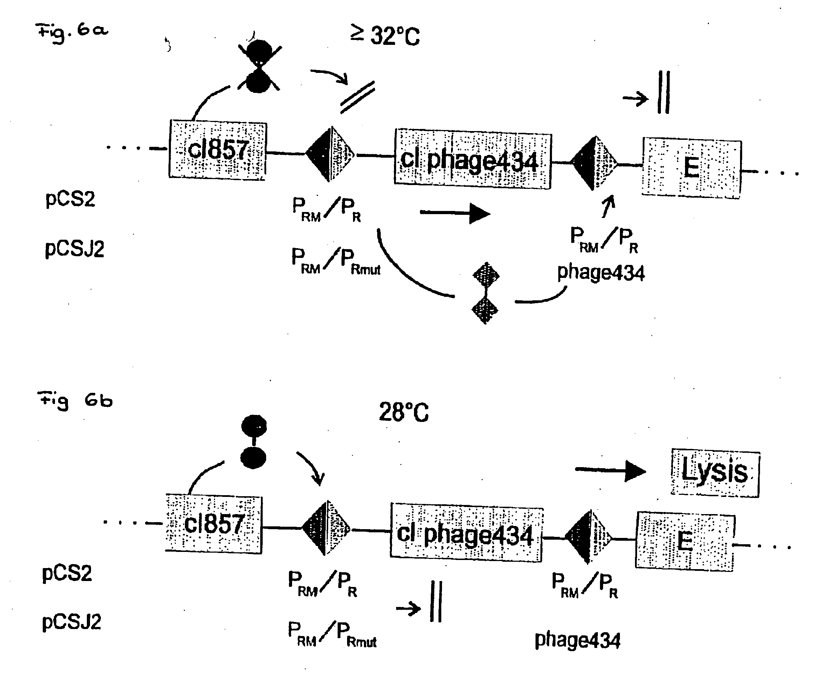

Fig. 6 zeigt eine weitere kältesensitive Sicherheitskassette. Die Plasmide pCS2 (Wildtyp-Operator) und pCSJ2 (mutierter Operator) zeigen bei Temperaturen, bei denen der Lambda cI857-Repressor nicht an den Operator bindet, die Bildung von cI-434 Repressormolekülen, die die Expression des E-Gens reprimieren (Fig. 6a). Bei einer Temperatur, bei der der cI857-Repressor an den Lambda-Operator bindet, wird die Bildung von cI-434-spezifischen Repressormolekülen unterbunden und so die Expression des E-Gens freigegeben (Fig. 6b).

### Beispiel 3:

### In vivo Analyse von kältesensitiven Lysekassetten

Es wurde die Abtötung von Bakterien durch Temperaturerniedrigung nach Passage durch einen Mäusedarm und Freisetzung durch Kot bestimmt.

Hierzu wurden jeweils einmal 10¹⁰ E.coli Bakterien Balb/c-Mäusen verabreicht und die im Kot freigesetzte Anzahl von Bakterien bestimmt. Die Auswertung erfolgte auf E.coli-spezifischen Endoplatten (Endo, Zentralbl. Bakt. I Orig. 35 (1904) 109-110) mit Tetrazyklin als Marker für die eingesetzten Plasmide. Die Inkubation erfolgte bei 28°C.

### Ergebnis :

Gegenüber einer Kontrolle aus E.coli NM522 (pAWJ-lac) kommt es bei den Versuchsgruppen E.coli NM522 (pCS2), E.coli MC4100 (pCS1) und E.coli MC4100 (pCSJ1) zu einer Keimzahlreduktion von mindestens 99,9 %, 98% und 80% gemessen 10 h und 20 h nach Verabreichung der E.coli Bakterien.

## Patentansprüche

1. Verfahren zur Selektion von mutierten O_{R}- oder O_{L}-Operator-DNA-Sequenzen aus lambdoiden Phagen, die eine im Vergleich zur Wildtypsequenz erhöhte Thermostabilität hinsichtlich der Bindung eines temperatursensitiven Repressors aufweisen,
**dadurch gekennzeichnet,**
**dass** man
(a) eine DNA-Kassette herstellt, die ein Selektionsgen unter operativer Kontrolle einer Expressionskontrollsequenz, umfassend mindestens eine O_{R}- oder O_{L}-Operatorsequenz aus einem lambdoiden Phagen und einem Promotor enthält,
(b) die Operator-DNA-Sequenz einer Mutagenese unterzieht,
(c) eine Selektion der mutierten Operator-DNA-Sequenzen durchführt, die eine im Vergleich zur Wildtypsequenz erhöhte Thermostabilität hinsichtlich der Bindung eines temperatursensitiven Repressors aufweisen, und
(d) die mutierten Operator-DNA-Sequenzen analysiert.

2. Verfahren nach Anspruch 1,
**dadurch gekennzeichnet,**
**dass** man eine Operator-DNA-Sequenz aus den Operatorregionen O_{R} oder/und O_{L} des Phagen Lambda verwendet.

3. Verfahren nach Anspruch 1 oder 2,
**dadurch gekennzeichnet,**
**dass** man als Selektionsgen das E-Lysegen aus dem Phagen PhiX174 verwendet.

4. Verfahren nach einem der Ansprüche 1-3,
**dadurch gekennzeichnet,**
**dass** man die Operator-DNA-Sequenz einer ortsspezifischen Mutagenese durch Oligonukleotide unterzieht oder eine Selektion in einem Mutator-Bakterienstamm durchführt.

5. Verfahren nach einem der Ansprüche 1-4,
**dadurch gekennzeichnet,**
**dass** die Analyse der mutierten Operator-DNA-Sequenzen durch Bestimmung der Bindefähigkeit mit einem temperatursensitiven cl-Repressor erfolgt.

6. Mutierte Lambda-O_{R}-Operatorsequenz, umfassend die in SEQ ID NO.2 gezeigte Sequenz.

7. Verfahren nach einem der Ansprüche 1-5,
**dadurch gekennzeichnet,**
**dass** die selektierten mutierten O_{R}- oder O_{L}-Operatorsequenzen zur temperaturregulierten Expression von Genen in Bakterienzellen verwendet werden.

8. Verfahren nach einem der Ansprüche 1-5,
**dadurch gekennzeichnet,**
**dass** eine Kombination von (a) einer Wildtyp-O_{R}- oder O_{L}-Operatorregion und mindestens einer Operatorregion, die eine selektierte mutierte O_{R}- oder O_{L}-Operatorsequenz enthält, oder (b) mehreren Operatorregionen, die selektierte mutierte O_{R}- oder O_{L}-Operatorsequenzen mit unterschiedlicher Thermostabilität enthalten, zur temperaturregulierten sequentiellen Expression von Genen verwendet wird.

9. Verfahren nach Anspruch 7 oder 8,
**dadurch gekennzeichnet,**
**dass** die Bakterienzellen zur Regulation der Genexpression ein Gen für einen cl-Repressor aus lambdoiden Phagen, insbesondere das Gen für den Lambda-cL857-Repressor enthalten.

10. Nukleinsäure, umfassend eine bakterielle Expressionskontrollsequenz, die eine mutierte O_{R}- oder O_{L}-Operatorsequenz nach Anspruch 6 enthält, in operativer Verknüpfung mit einer Protein-kodierenden Sequenz.

11. Nukleinsäure nach Anspruch 10,
**dadurch gekennzeichnet,**
**dass** die Protein-kodierende Sequenz ein Suizidgen ist.

12. Vektor,
**dadurch gekennzeichnet,**
**dass** er mindestens eine Kopie einer Nukleinsäure nach Anspruch 10 oder 11 enthält.

13. Bakterienzelle,
**dadurch gekennzeichnet,**
**dass** sie eine Nukleinsäure nach einem der Ansprüche 10-11 oder einen Vektor nach Anspruch 12 enthält.

14. Bakterienzelle nach Anspruch 13,
**dadurch gekennzeichnet,**
**dass** sie weiterhin ein Gen für einen cl-Repressor aus lambdoiden Phagen, insbesondere das Gen für den Lambda c1857 Repressor enthält.

15. Impfstoffzusammensetzung,
**dadurch gekennzeichnet,**
**dass** sie eine lebende Bakterienzelle nach Anspruch 13 oder 14 als Wirkstoff gegebenenfalls mit pharmazeutisch verträglichen Hilfs-, Zusatz- und Trägerstoffen enthält.

16. Impfstoffzusammensetzung,
**dadurch gekennzeichnet,**
**dass** sie einen Bakterienghost als Wirkstoff gegebenenfalls mit pharmazeutisch verträglichen Hilfs-, Zusatz- und Trägerstoffen enthält, wobei der Bakterienghost durch Kultivierung einer Bakterienzelle nach einem der Ansprüche 13-14 bei Temperaturen von 35-39 °C und anschließende Lyse der Bakterienzelle durch Temperaturerhöhung erhältlich ist.

17. Nukleinsäure, umfassend (a) eine erste bakterielle Expressionskontrollsequenz, die eine mutierte O_{R}- oder O_{L}-Operatorsequenz aus einem lambdoiden Phagen nach Anspruch 6 enthält und an die ein erster cl-Repressor aus lambdoiden Phagen binden kann, in operativer Verknüpfung mit einer für einen zweiten Repressor kodierenden Sequenz, wobei der zweite Repressor nicht an die erste bakterielle Expressionskontrollsequenz binden kann, und (b) eine zweite bakterielle Expressionskontrollsequenz, an die der zweite Repressor binden kann, in operativer Verknüpfung mit einem Suizidgen.

18. Bakterienzelle,
**dadurch gekennzeichnet,**
**dass** sie mindestens eine Kopie einer Nukleinsäure nach Anspruch 17 enthält.

19. Bakterienzelle nach Anspruch 18, weiterhin umfassend (c) eine dritte bakterielle Expressionskontrollsequenz, die eine mutierte Operatorsequenz nach Anspruch 6 enthält, in operativer Verknüpfung mit einem Suizidgen.

20. Impfstoffzusammensetzung,
**dadurch gekennzeichnet,**
**dass** sie eine lebende Bakterienzelle nach Anspruch 18 oder 19 als Wirkstoff gegebenenfalls mit pharmazeutisch verträglichen Hilfs-, Zusatz- und Trägerstoffen enthält.

21. Verwendung von Impfstoffzusammensetzungen nach Anspruch 15 oder 20 als wärme- oder/und kälteempfindliche Sicherheitslebendvakzine.

## Claims

1. Method for selecting mutated O_{R} or O_{L} operator DNA sequences from lambdoid phages which have an increased thermostability compared to the wild-type sequence with regard to the binding of a temperature-sensitive repressor,
**characterized in that**
(a) a DNA cassette is prepared which contains a selection gene under the operative control of an expression control sequence comprising at least one O_{R} or O_{L} operator sequence from a lambdoid phage and a promoter,
(b) the operator DNA sequence is subjected to a mutagenesis,
(c) the mutated operator DNA sequences are selected which have an increased thermostability compared to the wild-type sequence with regard to the binding of a temperature-sensitive repressor and
(d) the mutated operator DNA sequences are analysed.

2. Method according to claim 1,
**characterized in that**
an operator DNA sequence from the operator regions O_{R} or/and O_{L} of the phage lambda is used.

3. Method according to claim 1 or 2,
**characterized in that**
the E-lysis gene from the phage PhiX 174 is used as the selection gene.

4. Method according to one of the claims 1-3,
**characterized in that**
the operator DNA sequence is subjected to a site-specific mutagenesis by oligonucleotides or a selection is carried out in a mutator bacterial strain.

5. Method according to one of the claims 1-4,
**characterized in that**
the mutated operator DNA sequences are analysed by determining their ability to bind to a temperature-sensitive cI repressor.

6. Mutated lambda O_{R} operator sequence comprising the sequence shown in SEQ ID No.2.

7. Method according to one of the claims 1 - 5,
**characterized in that**
the selected mutated O_{R} or O_{L} operator sequences are used for the temperature-regulated expression of genes in bacterial cells.

8. Method according to one of the claims 1-5,
**characterized in that**
a combination of (a) a wild-type O_{R} or O_{L} operator region and at least one operator region which contains a selected mutated O_{R} or O_{L} operator sequence or (b) several operator regions which contain selected mutated O_{R} or O_{L} operator sequences with different thermostabilities is used for the temperature-regulated sequential expression of genes.

9. Method according to claim 7 or 8,
**characterized in that**
the bacterial cells contain a gene for a cI repressor from lambdoid phages and in particular the gene for the lambda cI857 repressor for the regulation of gene expression.

10. Nucleic acid comprising a bacterial expression control sequence which contains a mutated O_{R} or O_{L} operator sequence according to claim 6 in operative linkage with a protein-coding sequence.

11. Nucleic acid according to claim 10,
**characterized in that**
the protein-coding sequence is a suicide gene.

12. Vector,
**characterized in that**
it contains at least one copy of a nucleic acid according to claim 10 or 11.

13. Bacterial cell,
**characterized in that**
it contains a nucleic acid according to one of the claims 10 - 11 or a vector according to claim 12.

14. Bacterial cell according to claim 13,
**characterized in that**
it additionally contains a gene for a cI repressor from lambdoid phages and in particular the gene for the lambda cI857 repressor.

15. Vaccine composition,
**characterized in that**
it contains a live bacterial cell according to claim 13 or 14 as an active ingredient optionally with pharmaceutically acceptable auxiliary substances, additives and carrier substances.

16. Vaccine composition,
**characterized in that**
it contains a bacterial ghost as the active ingredient optionally with pharmaceutically acceptable auxiliary substances, additives and carrier substances wherein the bacterial ghost can be obtained by culturing a bacterial cell according to one of the claims 13 -14 at temperatures of 35 - 39°C and subsequently lysing the bacterial cell by increasing the temperature.

17. Nucleic acid comprising (a) a first bacterial expression control sequence which contains a mutated O_{R} or O_{L} operator sequence from a lambdoid phage according to claim 6 and to which a first cI repressor from lambdoid phages can bind, in operative linkage with a sequence coding for a second repressor wherein the second repressor cannot bind to the first bacterial expression control sequence and (b) a second bacterial expression control sequence to which the second repressor can bind in operative linkage with a suicide gene.

18. Bacterial cell,
**characterized in that**
it contains at least one copy of a nucleic acid according to claim 17.

19. Bacterial cell according to claim 18, additionally comprising (c) a third bacterial expression control sequence which contains a mutated operator sequence according to claim 6 in operative linkage with a suicide gene.

20. Vaccine composition,
**characterized in that**
it contains a live bacterial cell according to claim 18 or 19 as the active ingredient optionally together with pharmaceutically acceptable auxiliary substances, additives and carrier substances.

21. Use of vaccine compositions according to claim 15 or 20 as heat-sensitive or/ and cold-sensitive safe live vaccines.

## Revendications

1. Procédé de sélection de séquences d'ADN opérateur O_{R} ou O_{L} mutées issues de phages lambdoïdes, qui présentent, par rapport à la séquence de type sauvage, une thermostabilité accrue concernant la liaison d'un répresseur sensible à la température, **caractérisé en ce que**
(a) on produit une cassette d'ADN qui contient un gène de sélection sous le contrôle fonctionnel d'une séquence de contrôle de l'expression, comprenant au moins une séquence opérateur O_{R} ou O_{L} issue d'un phage lambdoïde et un promoteur,
(b) on soumet la séquence d'ADN opérateur à une mutagenèse,
(c) on réalise une sélection des séquences d'ADN opérateur mutées qui présentent, par rapport à la séquence de type sauvage, une thermostabilité accrue concernant la liaison d'un répresseur sensible à la température, et
(d) on analyse les séquences d'ADN opérateur mutées.

2. Procédé selon la revendication 1 **caractérisé en ce que** l'on utilise une séquence d'ADN opérateur issue des régions opérateur O_{R} et/ou O_{L} du phage lambda.

3. Procédé selon la revendication 1 ou 2 **caractérisé en ce que** l'on utilise comme gène de sélection le gène de lyse E issu du phage PhiX174.

4. Procédé selon l'une des revendications 1-3 **caractérisé en ce qu'**on soumet la séquence d'ADN opérateur à une mutagenèse spécifique de site par oligonucléotide ou on réalise une sélection dans une souche bactérienne mutatrice.

5. Procédé selon l'une des revendications 1-4 **caractérisé en ce que** l'analyse des séquences d'ADN opérateur mutées a lieu par détermination de la capacité de liaison avec un répresseur cl sensible à la température.

6. Séquence opérateur O_{R} de lambda mutée comprenant la séquence montrée dans SEQ ID NO.2.

7. Procédé selon l'une des revendications 1-5 **caractérisé en ce que** les séquences d'opérateur O_{R} ou O_{L} mutées sélectionnées sont utilisées pour l'expression régulée par la température de gènes dans des cellules bactériennes.

8. Procédé selon l'une des revendications 1-5 **caractérisé en ce qu'**une combinaison de (a) une région opérateur O_{R} ou O_{L} de type sauvage et d'au moins une région opérateur qui contient une séquence opérateur O_{R} ou O_{L} mutée sélectionnée, ou de (b) plusieurs régions opérateur qui contiennent des séquences opérateur O_{R} ou O_{L} mutées sélectionnées ayant une thermostabilité différente est utilisée, pour l'expression séquentielle de gènes régulée par la température.

9. Procédé selon la revendication 7 ou 8 **caractérisé en ce que** les cellules bactériennes contiennent pour la régulation de l'expression génique un gène pour un répresseur cl issu de phages lambdoïdes, en particulier le gène pour le répresseur cL857 de lambda.

10. Acide nucléique comprenant une séquence de contrôle de l'expression bactérienne qui contient une séquence opérateur O_{R} ou O_{L} mutée selon la revendication 6 en liaison fonctionnelle avec une séquence codant une protéine.

11. Acide nucléique selon la revendication 10 **caractérisé en ce que** la séquence codant une protéine est un gène suicide.

12. Vecteur **caractérisé en ce qu'**il contient au moins une copie d'un acide nucléique selon la revendication 10 ou 11.

13. Cellule bactérienne **caractérisée en ce qu'**elle contient un acide nucléique selon l'une des revendications 10-11 ou un vecteur selon la revendication 12.

14. Cellule bactérienne selon la revendication 13 **caractérisée en ce qu'**elle contient en outre un gène pour un répresseur cl issu de phages lambdoïdes, en particulier le gène pour le répresseur cl 857 de lambda.

15. Composition vaccinale **caractérisée en ce qu'**elle contient une cellule bactérienne vivante selon la revendication 13 ou 14 comme principe actif éventuellement avec des adjuvants, additifs et vecteurs pharmaceutiquement acceptables.

16. Composition vaccinale **caractérisée en ce qu'**elle contient un ghost bactérien comme principe actif éventuellement avec des adjuvants, additifs et vecteurs pharmaceutiquement acceptables, où le ghost bactérien peut être obtenu par culture d'une cellule bactérienne selon l'une des revendications 13-14 à des températures de 35-39°C et lyse subséquente de la cellule bactérienne par élévation de la température.

17. Acide nucléique comprenant (a) une première séquence de contrôle de l'expression bactérienne, qui contient une séquence opérateur O_{R} ou O_{L} mutée issue d'un phage lambdoïde selon la revendication 6 et à laquelle peut se lier un premier répresseur cl issu de phages lambdoïdes, en liaison fonctionnelle avec une séquence codant un second répresseur, où le second répresseur ne peut pas se lier à la première séquence de contrôle de l'expression bactérienne, et (b) une seconde séquence de contrôle de l'expression bactérienne à laquelle le second répresseur peut se lier, en liaison fonctionnelle avec un gène suicide.

18. Cellule bactérienne **caractérisée en ce qu'**elle contient au moins une copie d'un acide nucléique selon la revendication 17.

19. Cellule bactérienne selon la revendication 18 comprenant en outre (c) une troisième séquence de contrôle de l'expression bactérienne qui contient une séquence opérateur mutée selon la revendication 6 en liaison fonctionnelle avec un gène suicide.

20. Composition vaccinale **caractérisée en ce qu'**elle contient une cellule bactérienne vivante selon la revendication 18 ou 19 comme principe actif éventuellement avec des adjuvants, additifs et vecteurs pharmaceutiquement acceptables.

21. Utilisation de compositions vaccinales selon la revendication 15 ou 20 comme vaccins vivants de sécurité sensibles à la chaleur et/ou au froid.
